# EUROPEAN PATENT SPECIFICATION

(11) **EP 1 758 586 B1**
(45) Date of publication and mention of the grant of the patent: **03.08.2011**
(21) Application number: 05770876.0
(22) Date of filing: 15.06.2005
(51) Int. Cl.: A61K 31/57, A61K 31/593, A61K 47/44, A61K 47/10, A61K 9/12, A61P 17/00, A61P 17/06

(54) **COMPOSITION IN SPRAY FORM COMPRISING A COMBINATION OF A CORTICOID AND A VITAMIN D DERIVATIVE IN AN OILY PHASE**
ZUSAMMENSETZUNG IN SPRAYFORM MIT EINER KOMBINATION AUS EINEM KORTIKOID UND EINEM VITAMIN-D-DERIVAT IN EINER ÖLPHASE
COMPOSITION SOUS FORME DE SPRAY COMPRENANT UNE COMBINAISON D'UN CORTICOIDE ET D'UN DERIVE DE LA VITAMINE D DANS UNE PHASE HUILEUSE

(30) Priority: 17.06.2004 FR 0406611; 28.09.2004 US 950650 P
(43) Date of publication of application: 07.03.2007
(73) Proprietor: Galderma S.A., 6330 Cham (CH)
(72) Inventor: WILLCOX, Nathalie, F-06460 Saint Vallier de Thiey (FR); ORSONI, Sandrine, F-06210 Mandelieu (FR); FREDON, Laurent Chemin du Camouyer, F-06330 Roquefort les Pins (FR)
(74) Representative: Allab, Myriam
(86) International application number: PCT/EP2005/007972
(87) International publication number: WO 2005/123090

(56) References cited:
- EP-A- 0 998 943
- WO-A-00/64450
- WO-A-2004/112798
- WO-A1-2006/005845
- FR-A- 2 737 118
- FR-A- 2 738 745
- FR-A- 2 740 038
- US-A- 4 610 978
- LAMBA S ET AL: "Combination therapy with vitamin D analogues." BRITISH JOURNAL OF DERMATOLOGY, vol. 144, no. Supplement 58, April 2001 (2001-04), pages 27-32, XP002253887 ISSN: 0007-0963
- AUSTAD J ET AL: "Clobetasol propionate followed by calcipotriol is superior to calcipotriol alone in topical treatment of psoriasis." JOURNAL OF THE EUROPEAN ACADEMY OF DERMATOLOGY AND VENEREOLOGY: JEADV. NETHERLANDS JUL 1998, vol. 11, no. 1, July 1998 (1998-07), pages 19-24, XP001154650 ISSN: 0926-9959

## Description

The invention relates to an anhydrous composition in spray form comprising as pharmaceutical active the combination of clobetasol propionate and calcitriol and an oily phase in a physiologically acceptable medium, to the process for preparing it and to its use in dermatology.

The combination of active principles is not used conventionally in the treatment of dermatological ailments. The main difficulties encountered by the person skilled in the art when combining two active principles are the problems of chemical instability and the interactions to which the active principles may give rise when they are present within the same formulation.

Little treatment therefore exists combining calcitriol and a corticoid (Lamba, S. et al.; British Journal of Dermatology, vol. 144, supplement 58, 2001, pages 27-32). The reason for this is that vitamin D and its derivatives are instable in aqueous media and sensitive to acidic pH values, while the corticoids, and more particularly clobetasol propionate, are for their part sensitive to basic media. It was therefore not obvious for the person skilled in the art to combine and to stabilize a vitamin D-type active and a corticosteroid within a single composition.

Calcitriol is a vitamin D analogue which is used to regulate the level of calcium in the body. Its use in the treatment of dermatological diseases was described in particular in patent US 4,610,978 for the treatment of psoriasis. That patent suggests compositions combining calcitriol which may further comprise an amount of an anti-inflammatory such as a corticosteroid, but no specific embodiment combining calcitriol and corticosteroid is either described or tested for its efficacy.

The applicant described in application FR 2 848 454 how combining calcitriol with a corticosteroid makes it possible to obtain a synergistic effect in the treatment of certain dermatological ailments such as psoriasis, atopic dermatitis, contact dermatitis and seborrheic dermatitis, though without proposing stable pharmaceutical compositions combining the two actives.

Furthermore, in the field of dermatology and of the formulation of pharmaceutical compositions, the person skilled in the art is induced to look for compositions which not only must be stable physically and chemically but also must make it possible to release the active and to promote its penetration through the skin layers in order to enhance its efficacy.

Pharmaceutical compositions must further exhibit good cosmetic qualities and must be preferably non-irritant.

Numerous topical compositions are currently in existence which comprise an active agent and allow its penetration into the skin to be promoted by virtue of the presence in particular of a high content of glycol penetration promoter. These compositions are formulated in the form of emulsions with a high fatty phase content which are commonly referred to as "lipocreams", in the form of anhydrous compositions which are referred to as "ointments", in the form of fluid compositions with a high content of volatile solvents, such as ethanol or isopropanol, which are intended for application to the scalp, and are also called "hair lotions", or else in the form of viscous O/W emulsions, which are also called "O/W creams".

The stabilization of a formulation comprising such a percentage of glycol makes it necessary to employ in the emulsion emulsifiers and stabilizers of glyceryl stearate or PEG 100 stearate type or else stabilizers or consistency factors of white wax or cetostearyl alcohol type which lead to the formation of a viscous cream. This viscosity therefore makes the product difficult to apply. These compositions, however, exhibit on the one hand poor cosmetic acceptability owing to their viscosity and, on the other hand, risks of intolerance, brought about by the presence of high proportions of glycol. These high viscosities, moreover, make the formulations difficult to apply to the various parts of the body affected by the pathology. Consequently the majority of existing treatments, in the form of creams or gels or ointments, necessitate the aid of a third person for their application to areas which are difficult to reach. The third person must therefore touch both the product containing the active and the psoriatic plaques, leading to a situation which is less than ideal from the standpoint of convenience of use and the third person's safety. The person skilled in the art is also aware that non-compliance with the prescribed treatment for the reasons set out above is one of the principal causes of failure; the article "Patients with psoriasis and their compliance with medication" (Richard & all, J Am Acad Dermatol Oct 99, p581-583) indicates that almost 40% of patients with a chronic disease such as psoriasis do not follow their treatment. It has been demonstrated that compliance by the patient with his or her treatment is directly linked to the characteristics of the vehicle of the applied composition. The article "Patients with psoriasis prefer solution and foam vehicles: a quantitative assessment of vehicle preference" (Housman & all; CUTIS, Dec 2002 vol 70, p327 to 332) indicates that psoriasis patients will give preference to a solution or a foam rather than to an unguent, cream or gel.

The person skilled in the art therefore wishes to improve these parameters by means of the present invention.

The closest prior art to the invention is that of international application WO 00/64450, which mentions the use of a pharmaceutical composition containing a vitamin D analogue and a corticosteroid. All of the composition examples of that patent application combine solely calcipotriol and beta-methasone dipropionate. The preferred compositions described in the application that allow the two actives to be stabilized are compositions in unguent form. These compositions therefore exhibit the drawbacks referred to above as far as convenience and ease of application are concerned. A reading of this prior art in any case does not allow the person skilled in the art to infer sprayable compositions which hence are easy to apply, as described in the present application, with the actives, clobetasol propionate and calcitriol, solubilized and stable within the composition.

This is because, from a reading of the prior art, either the existing treatments contain a high percentage of petroleum jelly, in order to promote occlusiveness and the penetration of the active, but have the disadvantage of being very greasy and sticky, or the compositions contain a high percentage of glycol penetration promoter in order to promote the penetration of the active, but are sticky and may give rise to problems of intolerance ("The critical role of the vehicle to therapeutic efficacy and patient compliance" Piacquadio & all, Journal of American Academy of dermatology, August 1998).

The problem which the present invention proposes solving is therefore to design a physically and chemically stable composition which allows the two actives calcitriol and clobetasol propionate, acting in synergy for the treatment of psoriasis, to be combined within a single composition, it being necessary also for the composition according to the invention to be easy to use and to have acceptable cosmetic properties for application to all of the areas of the body that may be affected by the pathology.

The term "physical stability" according to the invention refers to a composition which shows no change in its macroscopic appearance (phase separation, change in apparent colour, etc.) or in its microscopic appearance (recrystallization of the actives) after storage at temperatures of 4°C and 40°C for 2, 4, 8 and 12 weeks.

The term "chemical stability" according to the invention refers to a composition in which the active principle content remains stable after three months at ambient temperature and at 40°C. A stable active principle content signifies according to the invention that the content exhibits very little change relative to the initial content, in other words that the variation in active principle content at time T must not be less than 90%, more particularly 95% of the initial content at T0.

The applicant has found surprisingly that the composition comprising in a pharmaceutically acceptable vehicle
a) a therapeutically effective amount of clobetasol propionate in solubilized form ;
b) a therapeutically effective amount of calcitriol in solubilized form ;
c) an oily phase composed of one or more oils selected from the group consisting of caprylic/capric triglycerides, cetearyl isononanoate, cyclomethicones, dimethicones and sweet almond oil, the said composition being in anhydrous spray form, gave a composition which solves the said problems.

The composition of the present invention is stable chemically and physically while allowing effective penetration of the active principles. It also exhibits very good acceptability and tolerance on the part of patients, owing to its spray formula, as described in the examples of the present invention. It therefore turns out that the composition according to the invention is particularly suitable for the treatment of dermatological ailments and more particularly is highly suited to the treatment of psoriasis.

The invention accordingly provides a composition which is liquid at ambient temperature and sprayable, comprising in a pharmaceutically acceptable vehicle
a) a therapeutically effective amount of clobetasol propionate in solubilized form;
b) a therapeutically effective amount of calcitriol in solubilized form;
c) an oily phase composed of one or more oils selected from the group consisting of caprylic/capric triglycerides, cetearyl isononanoate, cyclomethicones, dimethicones and sweet almond oil.

By solubilized form is meant a dispersion in the molecular state in a liquid, with no crystallization of the active being visible to the naked eye nor to an optical microscope in crossed polarization.

A sprayable composition is any composition which is liquid and fluid and which flows rapidly under its own weight at ambient temperature. By ambient temperature is meant a temperature of approximately 25°C.

The spray may be obtained by conventional formulating means which are known to the skilled person, as explained hereinafter.

The composition is preferably anhydrous. An anhydrous composition in the sense of the present invention is a composition which is substantially free of water, i.e., which has a water content of less than or equal to 1% by weight relative to the total weight of the composition, in particular less than or equal to 0.5% and preferably equal to zero.

The composition according to the invention contains between 0.00001 and 0.1% by weight, relative to the total weight of the composition, of calcitriol. preferably between 0.0001 and 0.001% by weight and more preferably between 0.0002 and 0.0005% by weight. The composition according to the invention contains more particularly 0.0003% of calcitriol by weight, relative to the total weight of the composition.

The composition according to the invention contains between 0.0001 and 0.1% by weight, relative to the total weight of the composition, of clobetasol propionate, preferably between 0.001 and 0.05% by weight. Preferred compositions according to the invention contain more particularly 0.01%, 0.025% or 0.05% of clobetasol propionate by weight, relative to the total weight of the composition.

An oily phase according to the invention is an oily phase suitable for a pharmaceutical composition.

The oily phase according to the present invention plays a part, among others, as a solubilizer for the actives (and is also called solvent for actives). The oils are the only solvents for actives that can be used according to the present invention. Accordingly, in particular, alcoholic and glycolic solvents are excluded from the present invention.

The oily form is ideal for the psoriasis pathology and similar to a cosmetic massage oil. This liquid oily form makes it possible to provide the patient with the comfort of emollience without the drawbacks of applying a thick, very sticky and greasy ointment.

The selection and the ratio of the mixture of oils are determined as a function of their spreading powers and their chemical qualities. The selection of the oils which can be used in accordance with the invention is made such that the mixture thereof is clear and stable over time.

The compounds constituting the oily phase of the composition according to the invention are caprylic/capric triglycerides, sold under the name Miglyol 812, cetearyl isononanoate, sold under the name Cetiol SN, cyclomethicone 5, sold under the name Mirasil CM5, dimethicone 200 with a viscosity of 20 cst, and sweet almond oil, which are used alone or in a mixture.

The reasons for selecting these preferred compounds are as follows:

### - Selection of caprylic/capric triglycerides

Triglycerides are one of the components of the skin, forming part of the natural skin lipids together with the ceramides, cholesterol and the phospholipids. They are integrated into the deep layers of the epidermis and they compensate the skin's moisture loss. The protective barrier of the skin is regenerated specifically and durably.
The "medium chain triglycerides", of which Miglyol 812 is one, are composed of caprylic (C8) and capric (C10) fatty acids, which are derived from coconut oil or palm kernel oil.

The principal properties thereof are:
- low-viscosity emollient, increasing spreading on the skin;
- solvent for lipophilic active, rapidly penetrating the skin and promoting penetration of the active;
- absence of greasy sensation on application, without leaving greasy residues.

### - Selection of cetaryl isononanoate

Cetearyl isononanoate is an ester which has the particular feature of presenting a dry and soft feel to the skin.

### - Selection of cyclomethicone 5

Cyclomethicone 5 is a volatile silicone oil which allows easy application to the skin and leaves a relatively dry feeling after application.

### - Selection of dimethicone 200 with a viscosity of 20 cst

Dimethicone 200 is a silicone oil which allows easy application to the skin and leaves a non-greasy tactile feeling after application.

### - Selection of sweet almond oil

Sweet almond oil is a vegetable oil which is used for its softening properties.

The judicious mixing and selection of the oils make it possible to obtain a product which is totally oily but is much less greasy and sticky than unguents or ointments.

This also allows the patient to apply the product in sprayed form and allows, if desired, massage of the region to be treated, in contrast to a highly volatile sprayed product.

Advantageously the composition according to the invention contains between 50 and 99% by weight, relative to the total weight, of oily phase, preferably between 70 and 99% by weight and more preferably between 95 and 99% by weight.

The invention accordingly provides a sprayable composition comprising in a pharmaceutically acceptable vehicle
a) between 0.0001 and 0.1% of clobetasol propionate;
b) between 0.00001 and 0.1% of calcitriol;
c) between 50 and 99% of an oily phase composed of one or more oils selected from caprylic/capric triglycerides, cetearyl isononanoate, cyclomethicones, dimethicones, and sweet almond oil.

More particularly the sprayable composition preferred according to the present invention comprises in a pharmaceutically acceptable vehicle
a) between 0.001 and 0.05% of clobetasol propionate;
b) between 0.0002 and 0.0005% of calcitriol;
c) between 95 and 99% of an oily phase composed of one or more oils selected from caprylic/capric triglycerides, cetearyl isononanoate and cyclomethicones, dimethicones, and sweet almond oil.

According to one preferred embodiment the composition according to the invention likewise comprises antioxidant compounds such as DL-α-tocopherol, butylated hydroxyanisole or butylated hydroxytoluene, superoxide dismutase, ubiquinol or certain metal chelating agents. The antioxidants preferably used in the composition according to the invention are DL-α-tocopherol, butylated hydroxyanisole and butylated hydroxytoluene.

The composition according to the invention may likewise comprise surfactants. The surfactants which can be used according to the invention are of anionic surfactant type, such as carboxylates, and particularly soaps, alkylarylsulphonates, alkyl ether sulphates, alkyl sulphates and alcohol sulphates. More particularly the anions of these surfactants are coupled to a cation such as the metallic cations of sodium or of potassium. The preferred surfactants according to the invention are also surfactants of polysorbate and poloxamer type.
Preferably the surfactants used according to the present invention are sodium lauryl sulphate, polysorbate 80 (Tween 80 from Uniqema) and poloxamer 124 (Synperonic PEL44 from Uniqema).

The composition according to the invention may likewise comprise penetration promoters.

The pharmaceutical composition according to the invention might further comprise inert additives or combinations of these additives, such as:
- wetting agents;
- flavour enhancers;
- preservatives;
- stabilizers;
- moisture regulators;
- pH regulators;
- osmotic pressure modifiers;
- emulsifiers;
- UV-A and UV-B filters;
- penetration promoters; and
- synthetic polymers.

As will be appreciated, the person skilled in the art will take care that any compound or compounds for addition to these compositions will be selected such as not to alter, or substantially alter, the advantageous properties intrinsically attached to the present invention, as a result of the intended addition.

The composition according to the invention is more particularly intended for the treatment of the skin and the mucosae and is sprayable and suitable for packaging in spray form.
The spray exhibits numerous advantages over conventional forms, such as the ease with which the formula can be delivered into the areas of the body that are very difficult to treat, the possibility of ready control of the dose delivered, or the absence of contamination during use.

The composition according to the invention is therefore administered in the form of a sprayable composition. This composition may be obtained by conventional formulating means which are known to the person skilled in the art. For example the composition may be sprayed by a mechanical sprayer which pumps the composition within a container, flask or equivalent. Similarly the composition may be propelled by means of a gas, as is well known by the person skilled in the art. Conventional propellants such as air or hydrocarbons are effective provided that they do not interfere with the composition. The composition passes through a nozzle which may be pointed directly at the place where application is desired. The nozzle may be selected so as to apply the composition in the form of a vaporization or of a jet of droplets, according to the techniques known to the person skilled in the art. Depending on the pharmaceutical active selected, the spraying mechanism must be capable of always delivering the same amount of active. The mechanisms which allow the amount of composition to be delivered by the spray to be controlled are likewise known to the person skilled in the art. For example the amount of propellant may be calculated so as to propel the exact amount of product desired. For the composition according to the invention it is possible to use a metering vaporizer flask whose application surface characteristics and dose characteristics are controlled and reproducible. For example, the vaporizer may be composed of a flask fitted with a metering valve.

The composition of the present invention is chemically and physically stable while allowing effective penetration of the active principles. It also exhibits very good acceptability and tolerance on the part of patients, owing to its spray formula, as described in the examples of the present invention. It is therefore found that the composition according to the invention is particularly suitable for the treatment of dermatological ailments.

The present invention hence also provides for the use of a composition according to the invention for producing a medicinal product intended for the treatment of: - dermatological ailments with an inflammatory immunoallergic component, with or without a cell proliferation disorder, especially cutaneous psoriasis, mucosal psoriasis or ungual psoriasis, psoriatic rheumatism, cutaneous atopy and such as eczema.

In one preferred embodiment of the composition it will contain 0.01%, 0.025% or 0.05% of clobetasol 17-propionate and 0.0003% of calcitriol and will be used for producing a medicinal product intended for treating psoriasis.

The examples which follow show without limitation formulation examples of the composition according to the invention and also results for chemical and physical stability.

### Example 1 - Stability of calcitriol in various excipients

The example below describes the stability data for calcitriol in the various excipients preferred for the composition according to the invention, including caprylic/capric triglycerides and cetearyl isononanoate.

### a) Stability of calcitriol in Miglyol 812 (caprylic/capric triglycerides)

Solution of calcitriol 30 ppm in qs 100% of Miglyol 812 in the presence of 0.4% of BHT. HPLC assay technique against reference substance. At the starting time (T0) the composition is considered to contain 100% of calcitriol.

Concentration of calcitriol measured in % relative to T0:

| Stability conditions | T 2 weeks | T 4 weeks |
|---|---|---|
| +4°C | 98.3% | 105.2% |
| AT | 95.1% | 98.0% |
| +40°C | 91% | 93.8% |

### b) Stability of calcitriol in Cetiol SN (cetearyl isononanoate)

Solution of calcitriol 30 ppm in qs 100% of Cetiol SN (cetearyl isononanoate) in the presence of 0.4% of BHT.
HPLC assay technique against reference substance.
At the starting time (T0) the composition is considered to contain 100% of calcitriol.

Concentration of calcitriol measured in % relative to T0:

| Stability conditions | T 2 weeks | T 4 weeks |
|---|---|---|
| +4°C | 98.6% | 98.1% |
| AT | 98.7% | 98.4% |
| +40°C | 99.0% | 98.9% |

### Example 2 - Process for preparing compositions according to the invention

Compositions according to the present invention are prepared at ambient temperature, under a fume hood and in non-actinic light.

Introduce the antioxidant, the Calcitriol and the solvent oil into a flask.

Carry out stirring until the calcitriol is completely dissolved.

Then add the CLOBETASOL PROPIONATE.

Continue stirring until the clobetasol propionate is dissolved.

When the two actives are completely dissolved, introduce the remaining ingredients of the formula in succession.

Leave under stirring until the mixture is perfectly homogeneous.

### Example 3

| INGREDIENTS | % |
|---|---|
| CYCLOMETHICONE 5 | qs 100 |
| MEDIUM CHAIN TRIGLYCERIDES | 40 |
| CALCITRIOL | 0.0003 |
| CLOBETASOL 17-PROPIONATE | 0.025 |
| DL-ALPHA-TOCOPHEROL ACETATE | 1 |

The procedure is that described in Example 2. A colourless liquid solution is obtained.

### Example 4

| INGREDIENTS | % |
|---|---|
| CYCLOMETHICONE 5 | qs 100 |
| MEDIUM CHAIN TRIGLYCERIDES | 40 |
| CALCITRIOL | 0.0003 |
| CLOBETASOL 17-PROPIONATE | 0.025 |
| 1,2-PROPANEDIOL | 10 |
| ALMOND OIL | 5 |
| BUTYLATED HYDROXYTOLUENE | 0.04 |

The procedure is that described in Example 2. A very slightly yellow liquid solution is obtained.

### Example 5

| INGREDIENTS | % |
|---|---|
| CETEARYL ISONONANOATE | qs 100 |
| MEDIUM CHAIN TRIGLYCERIDES | 40 |
| CALCITRIOL | 0.0003 |
| CLOBETASOL 17-PROPIONATE | 0.025 |
| DIMETHICONE 200, 20 CST | 10 |
| ALMOND OIL | 5 |
| BUTYLATED HYDROXYTOLUENE | 0.04 |

The procedure is that described in Example 2. A very slightly yellow liquid solution is obtained.

### Example 6

| INGREDIENTS | % |
|---|---|
| MEDIUM CHAIN TRIGLYCERIDES | qs 100 |
| CYCLOMETHICONE 5 | 45 |
| CALCITRIOL | 0.0003 |
| CLOBETASOL 17-PROPIONATE | 0.025 |
| DIMETHICONE 200, 20 CST | 10 |
| ALMOND OIL | 5 |
| BUTYLATED HYDROXYTOLUENE | 0.04 |

The procedure is that described in Example 2. A very slightly yellow liquid solution is obtained.

### Example 7: Physical stability of the composition according to Example 6

The physical stability of the formulations is measured by macroscopic and microscopic observation of the formulation at ambient temperature, at 4°C and at 40°C after 2, 4, 8 and 12 weeks.

At ambient temperature macroscopic observation allows the physical integrity of the products to be guaranteed and microscopic observation makes it possible to verify that there is no recrystallization of the solubilized active.

At 4°C microscopic observation verifies the non-recrystallization of the solubilized actives.

At 40°C macroscopic observation verifies the integrity of the end product.

### Specifications at T0

*Macroscopic appearance*: Colourless or very slightly yellow liquid spray.

*Microscopic appearance*: Absence of crystals of calcitriol and of clobetasol 17-propionate.

| Time→ Stability conditions↓ | T 1M | T 2M | T 3M |
|---|---|---|---|
| AT | Conforms to specifications | Conforms to specifications | Conforms to specifications |
| +4°C | Conforms to specifications | Conforms to specifications | Conforms to specifications |
| +40°C | Conforms to specifications | Conforms to specifications | Conforms to specifications |

### Example 8: Chemical stability of the actives within the composition according to Example 6

### - Stability of the CALCITRIOL

The active is assayed by internal calibration in HPLC.

| Time→ Stability conditions↓ | T 0 | T 15d | T 1M | T 2M | T 3M |
|---|---|---|---|---|---|
| AT | 104.4% | 100.9% | 101.9% | 103.2% | 105.1% |
| +40°C | | 110.2% | 101% | 105.4% | 104.9% |

### - Stability of the clobetasol 17-propionate

Assay of the active by internal calibration in HPLC.

| Time→ Stability conditions↓ | T 0 | T 15d | T 1M | T 2M | T 3M |
|---|---|---|---|---|---|
| AT | 103.8% | 102.0% | 105.1% | 104.4% | 102.7% |
| +40°C | | 102.3% | 105.3% | 104.5% | 102.6% |

### Example 9

| INGREDIENTS | % |
|---|---|
| MEDIUM CHAIN TRIGLYCERIDES | qs 100 |
| CETEARYL ISONONANOATE | 45 |
| CALCITRIOL | 0.0003 |
| CLOBETASOL 17-PROPIONATE | 0.025 |
| ALMOND OIL | 5 |
| BUTYLATED HYDROXYTOLUENE | 0.04 |

The procedure is that described in Example 2. A very slightly yellow liquid solution is obtained.

### Example 10: Physical stability of the composition according to Example 9

The physical stability of the formulations is measured by macroscopic and microscopic observation of the formulation at ambient temperature, at 4°C and at 40°C after 2, 4, 8 and 12 weeks.
At ambient temperature macroscopic observation allows the physical integrity of the products to be guaranteed and microscopic observation makes it possible to verify that there is no recrystallization of the solubilized active.
At 4°C microscopic observation verifies the non-recrystallization of the solubilized actives.
At 40°C macroscopic observation verifies the integrity of the end product.

### Specifications at T0

*Macroscopic appearance*: Colourless or very slightly yellow liquid spray.

*Microscopic appearance*: Absence of crystals of calcitriol and of clobetasol 17-propionate.

| Time→ Stability conditions↓ | T 1M | T 2M | T 3M |
|---|---|---|---|
| AT | Conforms to specifications | Conforms to specifications | Conforms to specifications |
| +4°C | Conforms to specifications | Conforms to specifications | Conforms to specifications |
| +40°C | Conforms to specifications | Conforms to specifications | Conforms to specifications |

### Example 11: Chemical stability of the actives within the composition according to Example 9

### - Stability of the CALCITRIOL

The active is assayed by internal calibration in HPLC.

| Time→ Stability conditions↓ | T 0 | T 15d | T 1M | T 2M | T 3M |
|---|---|---|---|---|---|
| AT | 100.2% | 101.3% | 103.2% | 104.4% | 106.1% |
| +40°C | | 103.1% | 100.7% | 101.9% | 106% |

### - Stability of the clobetasol 17-propionate

Assay of the active by internal calibration in HPLC.

| Time→ Stability conditions↓ | T 0 | T 15d | T 1M | T 2M | T 3M |
|---|---|---|---|---|---|
| AT | 100.6% | 98.4% | 102.4% | 102.8% | 100.6% |
| +40°C | | 99.6% | 102.2% | 102.9% | 99.3% |

### Example 12

| INGREDIENTS | % |
|---|---|
| CYCLOMETHICONE 5 | qs 100 |
| MEDIUM CHAIN TRIGLYCERIDES | 45 |
| CALCITRIOL | 0.0003 |
| CLOBETASOL 17-PROPIONATE | 0.025 |
| DIMETHICONE 200, 20 CST | 10 |
| BUTYLATED HYDROXYTOLUENE | 0.04 |

The procedure is that described in Example 2.

A colourless liquid solution is obtained.

### Specifications at T0

*Macroscopic appearance*: Colourless or very slightly yellow liquid spray.

*Microscopic appearance*: Absence of crystals of calcitriol and of clobetasol 17-propionate.

### Example 13

| INGREDIENTS | % |
|---|---|
| MEDIUM CHAIN TRIGLYCERIDES | qs 100 |
| CYCLOMETHICONE 5 | 35 |
| CALCITRIOL | 0.0003 |
| CLOBETASOL 17-PROPIONATE | 0.025 |
| DIMETHICONE 200, 20 CST | 10 |
| ALMOND OIL | 5 |
| BUTYLATED HYDROXYTOLUENE | 0.04 |

The procedure is that described in Example 2.

A very slightly yellow liquid solution is obtained.

### Example 14: Physical stability of the composition according to Example 13

The physical stability of the formulations is measured by macroscopic and microscopic observation of the formulation at ambient temperature, at 4°C and at 40°C after 2, 4, 8 and 12 weeks.
At ambient temperature macroscopic observation allows the physical integrity of the products to be guaranteed and microscopic observation makes it possible to verify that there is no recrystallization of the solubilized active.
At 4°C microscopic observation verifies the non-recrystallization of the solubilized actives.
At 40°C macroscopic observation verifies the integrity of the end product.

### Specifications at T0

*Macroscopic appearance*: Colourless or very slightly yellow liquid spray.

*Microscopic appearance*: Absence of crystals of calcitriol and of clobetasol 17-propionate.

| Time↓ Stability conditions↓ | T 1M | T 2M | T 3M |
|---|---|---|---|
| AT | Conforms to specifications | Conforms to specifications | Conforms to specifications |
| +4°C | Conforms to specifications | Conforms to specifications | Conforms to specifications |
| +40°C | Conforms to specifications | Conforms to specifications | Conforms to specifications |

### Example 15: Chemical stability of the actives within the composition according to Example 12

### Stability of the CALCITRIOL

| Time→ Stability conditions↓ | T 0 | T 15d | T 1M | T 2M | T 3M |
|---|---|---|---|---|---|
| AT | 114.3% | 114.1% | 113.6% | 115.8% | 119.3% |
| +40°C | | 113.4% | 114.8% | 117.2% | 119% |

### Stability of the CLOBETASOL 17-PROPIONATE

| Time→ Stability conditions↓ | T 0 | T 15d | T 1M | T 2M | T 3M |
|---|---|---|---|---|---|
| AT | 98.8% | 100.5% | 103.2% | 102.6% | 100.3% |
| +40°C | | 100.8% | 103% | 102.1% | 99.2% |

### Example 16: Study of the liberation/penetration in vitro on human skin of the active clobetasol 17-propionate contained in 3 different formulations, including one according to the invention

The objective is to quantify the skin penetration of the formulated active in different formulations *in vitro* on human skin after 16 hours of application.

Formulations tested:
- Emollient cream Temovate^{®} containing 0.05% (w/w) of clobetasol 17-propionate
- Cream Temovate^{®} containing 0.05% (w/w) of clobetasol 17-propionate
- Composition according to the invention of formula A below:

| INGREDIENTS | % |
|---|---|
| MEDIUM CHAIN TRIGLYCERIDES | qs 100 |
| CETEARYL ISONONANOATE | 45 |
| CALCITRIOL | 0.0003 |
| CLOBETASOL 17-PROPIONATE | 0.05 |
| ALMOND OIL | 5 |
| BUTYLATED HYDROXYTOLUENE | 0.04 |

The emollient cream Temovate^{®} is sold by the company GlaxoSmithKline.

**Experimental conditions:** The percutaneous absorption is evaluated by means of diffusion cells consisting of two compartments which are separated by human skin. The formulations were applied without occlusion for an application time of 16 hours. The formulations were applied at a rate of 10 mg of formulation per cm² (*i.e.* 10 micrograms of clobetasol 17-propionate). Throughout the study the dermis is in contact with a receiving liquid which is not renewed as a function of time (static mode). The experiments were conducted with 3 samples of skin from 3 different donors. At the end of the application period, the surface excess is removed and the distribution of the clobetasol 17-propionate is quantified in the various compartments of the skin and in the receiving liquid. The concentrations of clobetasol 17-propionate were quantified using an HPLC/MS/MS method conventionally known to the skilled person. (LQ: 1 ng·ml⁻¹).

The results are expressed in % of the dose applied (mean +/- standard deviation) and are set out in the table below.

| Formulation | | **Total amount** **having penetrated** |
|---|---|---|
| Emollient | Mean | **5.00** |
| Temovate cream | SEM | **1.34** |
| Temovate cream | Mean | **8.43** |
| | SEM | **0.79** |
| Composition A | Mean | **9.87** |
| | SEM | **4.05** |

The results show that the amount of clobetasol having penetrated with the composition according to the invention is equivalent to that of the Temovate emollient cream.

## Claims

1. Composition comprising in a pharmaceutically acceptable vehicle
a) a therapeutically effective amount of clobetasol propionate in solubilized form;
b) a therapeutically effective amount of calcitriol in solubilized form;
c) an oily phase composed of one or more oils selected from the group consisting of caprylic/capric triglycerides, cetearyl isononanoate, cyclomethicones, dimethicones and sweet almond oil;
**characterized in that** the composition is liquid at ambient temperature and sprayable.

2. Composition according to Claim 1, **characterized in that** the corticoid and the vitamin D derivative are solubilized in the said oily phase.

3. Composition according to any one of Claims 1 to 2 comprising in a pharmaceutically acceptable vehicle
a) between 0.0001 and 0.1% of clobetasol propionate;
b) between 0.00001 and 0.1% of calcitriol;
c) between 50 and 99% of an oily phase composed of one or more oils selected from caprylic/capric triglycerides, cetearyl isononanoate, cyclomethicones, dimethicones and sweet almond oil.

4. Composition according to Claim 3 comprising in a pharmaceutically acceptable vehicle
a) between 0.001 and 0.05% of clobetasol propionate;
b) between 0.0002 and 0.0005% of calcitriol;
c) between 95 and 99% of an oily phase composed of one or more oils selected from caprylic/capric triglycerides, cetearyl isononanoate, cyclomethicones, dimethicones and sweet almond oil.

5. Composition according to any one of Claims 1 to 5, **characterized in that** it further comprises an antioxidant.

6. Composition according to Claim 5, **characterized in that** the antioxidant is selected from DL-α-tocopherol, butylated hydroxyanisole and butylated hydroxytoluene.

7. Composition according to any one of Claims 1 to 6, **characterized in that** it further comprises a surfactant.

8. Composition according to Claim 7, **characterized in that** the surfactant is selected from sodium lauryl sulphate, poloxamers and polysorbates.

9. Composition according to any one of Claims 1 to 8, **characterized in that** it further comprises a penetration promoter.

10. Use of a composition according to any q one of Claims 1 to 9 for producing a medicinal product intended for the treatment of:
- dermatological ailments with an inflammatory immunoallergic component, with or without a cell proliferation disorder, especially cutaneous psoriasis, mucosal psoriasis or ungual psoriasis, psoriatic rheumatism and cutaneous atopy, such as eczema.

11. Use of a composition, according to Claim 10, for treating psoriasis.

## Patentansprüche

1. Zusammensetzung, die in einem pharmazeutisch akzeptablen Träger enthält:
a) eine therapeutisch wirksame Menge des Clobetasolpropionats in solubilisierter Form;
b) eine therapeutisch wirksame Menge des Calcitriols in solubilisierter Form;
c) eine Ölphase, die aus einem oder mehreren Ölen zusammengesetzt ist, die unter Capryl/Caprinsäure-Triglyceriden, Cetearylisononanoat, Cyclomethiconen, Dimethiconen und Süßmandelöl ausgewählt sind,
**dadurch gekennzeichnet, dass** die Zusammensetzung bei Raumtemperatur flüssig und zerstäubbar ist.

2. Zusammensetzung nach Anspruch 1, **dadurch gekennzeichnet, dass** das Corticoid und das Vitamin-D-Derivat in der Ölphase solubilisiert sind.

3. Zusammensetzung nach einem der Ansprüche 1 bis 2, die in einem pharmazeutisch akzeptablen Träger enthält:
a) 0,0001 bis 0,1 % Clobetasolpropionat;
b) 0,00001 bis 0,1 % Calcitriol;
c) 50 bis 99 % einer Ölphase, die aus einem oder mehreren Ölen zusammengesetzt ist, die unter Capryl/Caprinsäure-Triglyceriden, Cetearylisononanoat, Cyclomethiconen, Dimethiconen und Süßmandelöl ausgewählt sind.

4. Zusammensetzung nach Anspruch 3, die in einem pharmazeutisch akzeptablen Träger enthält:
a) 0,001 bis 0,05 % Clobetasolpropionat;
b) 0,0002 bis 0,0005 % Calcitriol;
c) 95 bis 99 % einer Ölphase, die aus einem oder mehreren Ölen zusammengesetzt ist, die unter Capryl/Caprinsäure-Triglyceriden, Cetearylisononanoat, Cyclomethiconen, Dimethiconen und Süßmandelöl ausgewählt sind.

5. Zusammensetzung nach einem der Ansprüche 1 bis 5, **dadurch gekennzeichnet, dass** sie ferner ein Antioxidationsmittel enthält.

6. Zusammensetzung nach Anspruch 5, **dadurch gekennzeichnet, dass** das Antioxidationsmittel unter DL-α-Tocopherol, Butylhydroxyanisol und Butylhydroxytoluol ausgewählt ist.

7. Zusammensetzung nach einem der Ansprüche 1 bis 6, **dadurch gekennzeichnet, dass** sie ferner einen grenzflächenaktiven Stoff enthält.

8. Zusammensetzung nach Anspruch 7, **dadurch gekennzeichnet, dass** der grenzflächenaktive Stoff unter Natriumlaurylsulfat, Poloxameren und Polysorbaten ausgewählt ist.

9. Zusammensetzung nach einem der Ansprüche 1 bis 8, **dadurch gekennzeichnet, dass** sie ferner einen penetrationsfördernden Stoff enthält.

10. Verwendung einer Zusammensetzung nach einem der Ansprüche 1 bis 9 für die Herstellung eines Arzneimittels, das vorgesehen ist für die Behandlung von:
- dermatologischen Erkrankungen mit einer entzündlichen immunoallergischen Komponente, mit oder ohne Störung der Zellproliferation, insbesondere Psoriasis der Haut, Psoriasis der Schleimhaut oder Psoriasis des Nagels, Psoriasis-Arthritis und Atopie der Haut, wie Ekzemen.

11. Verwendung einer Zusammensetzung nach Anspruch 10 für die Behandlung von Psoriasis.

## Revendications

1. Composition comprenant dans un véhicule pharmaceutiquement acceptable
a) une quantité thérapeutiquement efficace de propionate de clobétasol sous forme solubilisée ;
b) une quantité thérapeutiquement efficace de calcitriol sous forme solubilisée ;
c) une phase huileuse composée d'une ou de plusieurs huiles choisies dans le groupe constitué par les triglycérides capryliques/capriques, l'isononanoate de cétéaryle, les cyclométhicones, les diméthicones et l'huile d'amande douce ;
**caractérisée en ce que** la composition est liquide à température ambiante et pulvérisable.

2. Composition selon la revendication 1, **caractérisée en ce que** le corticoïde et le dérivé de vitamine D sont solubilisés dans ladite phase huileuse.

3. Composition selon l'une quelconque des revendications 1 à 2, comprenant dans un véhicule pharmaceutiquement acceptable
a) entre 0,0001 et 0,1 % de propionate de clobétasol ;
b) entre 0,00001 et 0,1 % de calcitriol ;
c) entre 50 et 99 % d'une phase huileuse composée d'une ou de plusieurs huiles choisies parmi les triglycérides capryliques/capriques, l'isononanoate de cétéaryle, les cyclométhicones, les diméthicones et l'huile d'amande douce.

4. Composition selon la revendication 3, comprenant dans un véhicule pharmaceutiquement acceptable
a) entre 0,001 et 0,05 % de propionate de clobétasol ;
b) entre 0,0002 et 0,0005 % de calcitriol ;
c) entre 95 et 99 % d'une phase huileuse composée d'une ou de plusieurs huiles choisies parmi les triglycérides capryliques/capriques, l'isononanoate de cétéaryle, les cyclométhicones, les diméthicones et l'huile d'amande douce.

5. Composition selon l'une quelconque des revendications 1 à 4, **caractérisée en ce qu'**elle comprend également un antioxydant.

6. Composition selon la revendication 5, **caractérisée en ce que** l'antioxydant est choisi parmi le DL-α-tocophérol, l'hydroxyanisole butylé et l'hydroxytoluène butylé.

7. Composition selon l'une quelconque des revendications 1 à 6, **caractérisée en ce qu'**elle comprend également un tensioactif.

8. Composition selon la revendication 7, **caractérisée en ce que** le tensioactif est choisi parmi le laurylsulfate de sodium, les poloxamères et les polysorbates.

9. Composition selon l'une quelconque des revendications 1 à 8, **caractérisée en ce qu'**elle comprend également un promoteur de pénétration.

10. Utilisation d'une composition selon l'une quelconque des revendications 1 à 9 pour la fabrication d'un produit médical prévu pour le traitement de :
- maladies dermatologiques ayant un composant immunoallergique inflammatoire, avec ou sans trouble de la prolifération cellulaire, notamment le psoriasis cutané, le psoriasis des muqueuses ou le psoriasis des ongles, les rhumatismes psoriasiques et l'atopie cutanée, telle que l'eczéma.

11. Utilisation d'une composition selon la revendication 10, pour le traitement du psoriasis.
